# EUROPÄISCHE PATENTANMELDUNG

(11) **EP 1 063 218 A1**
(43) Veröffentlichungstag der Anmeldung: **27.12.2000**
(21) Anmeldenummer: 00111625.0
(22) Anmeldetag: 31.05.2000
(51) Int. Cl.: C07C 45/34, C07C 49/603

(54) **Verfahren zur Herstellung von Oxoisophoron**

(30) Priorität: 25.06.1999 DE 19929367
(71) Anmelder: BASF AKTIENGESELLSCHAFT, 67056 Ludwigshafen (DE)
(72) Erfinder: Klatt, Martin Jochen, Dr., 67098 Bad Dürkheim (DE); Müller, Thomas, Dr., 67246 Dirmstein (DE); Bockstiegel, Bernhard, Dr., 67354 Römerberg (DE)

(57) **Zusammenfassung**

Die vorliegende Erfindung betrifft ein Verfahren zur Herstellung von 3,5,5-Trimethylcyclohex-2-en-1,4-dion (Oxoisophoron; OIP) durch Oxidation von 3,5,5-Trimethyl-cyclohex-3-en-1-on (β-Isophoron, β-IP) mit molekularem Sauerstoff in Gegenwart eines Lösungsmittels, einer Base und einem Mangan-Salenderivat als Katalysator.

## Beschreibung

Die vorliegende Erfindung betrifft ein Verfahren zur Herstellung von 3,5,5-Trimethylcyclohex-2-en-1,4-dion (Oxoisophoron; OIP) durch Oxidation von 3,5,5-Trimethyl-cyclohex-3-en-1-on (β-Isophoron, β-IP) mit molekularem Sauerstoff in Gegenwart eines Lösungsmittels, einer Base und einem Mangan-Salenderivat als Katalysator.

Oxoisophoron (OIP) kann als Geschmacks- oder Riechstoff in Lebensmitteln oder in kosmetischen Formulierungen verwendet werden. OIP ist darüberhinaus ein Zwischenprodukt für die Herstellung von Vitaminen und Carotinoiden.

Es ist bekannt, OIP durch Oxidation von β-Isophoron (β-IP) mit molekularem Sauerstoff in Gegenwart eines inerten Lösungsmittels, einer Base und eines Mn- oder Co-Salenderivats herzustellen. Die Patentschrift DE 2610254 C2 beschreibt die Verwendung einer Vielzahl von Cobalt(II)- und Mangan(II)salen-Derivaten als Katalysatoren, wobei die salenartigen Chelatliganden aus einer Reihe von Diaminen und Hydroxycarbonylverbindungen hergestellt werden können. In der Liste der Hydroxycarbonylverbindungen werden neben vielen anderen auch halogenierte 2-Hydroxybenzaldehyde mit einem weit variierbaren Substitutionsmuster erwähnt. Mit diesem Verfahren werden insbesondere bei Verwendung von unsubstituierten aromatischen Mn- uns Co-Salenen (Liganden hergestellt aus verschiedenen Diaminen und 2-Hydroxy-benzaldehyd) oftmals nur geringe Umsätze, Ausbeuten und Selektivitäten erreicht. Eine Substitution am Aromaten mit elektronenziehenden Resten, wie die Einführung einer Nitro-Gruppe führt, wie in Beispiel 5 *loc. cit.* gezeigt, zu einer geringeren Ausbeute.

In JP 01090150 werden in einem analogen Verfahren aromatische Mangan(III)salen-Derivate mit einer weiten Variationsmöglichkeiten im Substitutionsmuster, den Substituenten, dem Gegenion und der Anzahl der C-Atome in der Aminbrücke als Katalysatoren beschrieben. Als bestes Ergebnis werden unter der Verwendung eines chlorierten Mn(III)salens mit X = Acetat als Gegenion bei geringen Eduktkonzentrationen Ausbeuten bis zu 90,7 % erreicht. Aufgrund der geringen Eduktkonzentrationen ist die Raum-Zeit-Ausbeute aber ebenfalls gering.

Bei allen Verfahren des Standes der Technik entstehen neben Hochsiedern, die in der Aufreinigung weniger stören, die folgenden, identifizierten Nebenprodukte IV (α-Isophoron), V, VI und VII, die eine Aufreinigung oder eine weitere chemische Umsetzung des OIP erschweren:

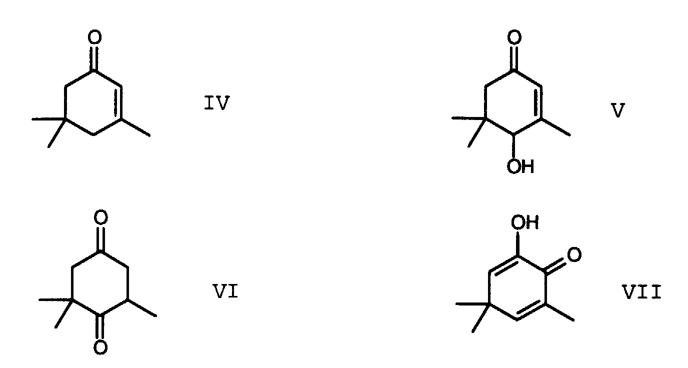

α-Isophoron (IV) entsteht bei den Verfahren nach dem Stand der Technik mit bis zu 3,2 % Ausbeute und Verbindung V mit bis zu 4,4 % Ausbeute. Besonders störend für weitere Synthesen ist die Verbindung VI, da sie ein ähnliches chemisches Verhalten zeigt wie das Produkt OIP.

Alle Verfahren des Standes der Technik haben den Nachteil, gemeinsam, bei steigender Eduktkonzentration nur geringe Ausbeuten und damit nur geringe Raum-Zeit-Ausbeuten an OIP zu liefern.

Alle bekannten Verfahren verwenden ferner aus der Vielzahl der Kombinationsmöglichkeiten an Basen und Lösungsmitteln Triethylamin als Base, mehrheitlich kombiniert mit Ethern wie Diglyme (Dimethyldiglykol) als Lösungsmittel. Da dieses Gemisch eine Zündtemperatur von 0°C aufweist, lassen sich die bekannten Verfahren auch aus diesem Grund nur unter hohem Sicherheitsaufwand im großtechnischen Maßstab durchführen.

Es stellte sich daher die Aufgabe, den geschilderten Mangeln abzuhelfen und ein Verfahren zur Oxidation von β-Isophoron mit Mangansalenkomplexen mit optimierten Eigenschaften zu entwickeln, das selbst bei hohen Eduktkonzentrationen gute Ausbeuten, Selektivitäten und Raum-Zeit-Ausbeuten liefert, die sich auch in den großtechnischen Maßstab übertragen lassen.

Demgemäß wurde ein Verfahren gefunden, indem man 3,5,5-Trimethylcyclohex-2-en-1,4-dion durch Oxidation von 3,5,5-Trimethylcyclohex-3-en-1-on mit molekularem Sauerstoff in Gegenwart eines Lösungsmittels, einer Base und einem Katalysator herstellt, dadurch gekennzeichnet, daß man als Katalysator einen Mangansalenkomplex der allgemeinen Formel I 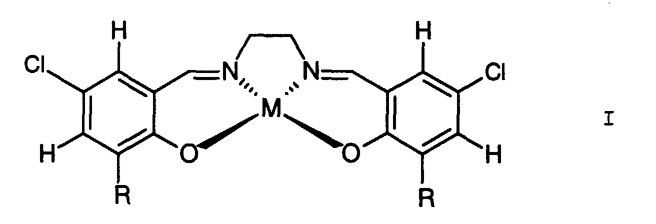 verwendet, wobei
- R: Wasserstoff oder Cl und
- M: Mn(II) oder Mn(III) ⁽⁺⁾Cl⁽⁻⁾ bedeuten.

Ausgangsverbindung des Verfahrens ist 3,5,5-Trimethyl-cyclohex-3-en-1-on (β-Isophoron; β-IP). Die Umsetzung zu 3,5,5-Trimethylcyclohex-2-en-1,4-dion (Oxoisophoron; OIP) erfolgt in einem Lösungsmittel durch Oxidation mit molekularem Sauerstoff in Gegenwart einer Base und dem vorstehend erwähnten Katalysator der Formel I.

Die Katalysatoren der Formel I sind zweifach bzw. vierfach mit Chlor substituierte Mangan-Salenderivate, die aus dem zentralen Manganatom in der Oxidationsstufe II oder III und dem entsprechenden, zweifach bzw. vierfach mit Chlor substituierten vier-zähnigen Chelatliganden (Salen-Liganden) aufgebaut sind. Der Salenligand kann in an sich bekannter Weise aus Ethylendiamin und 5-Chlor-2-Hydroxy-Benzaldehyd bzw. 3,5-Dichlor-2-Hydroxy-Benzaldehyd hergestellt werden. Als negativ geladenes Gegenion trägt das Mangan in der Oxidationsstufe III im Komplex ein Chloridion. Die Herstellung der Mangan(II)salenkomplexe der Formel I mit R = H oder Cl erfolgt in an sich bekannter Weise, beispielsweise durch Umsetzung der entsprechenden Salenliganden mit MnSO₄. Die Herstellung der Mangan(III)salenkomplexe der Formel I mit R = H oder Cl und Chlorid als Gegenion erfolgt in an sich bekannter Weise, beispielsweise durch Umsetzung der entsprechenden Salenliganden mit Mn(OAc)₃ und LiCl.

Unter Lösungsmittel werden organische Lösungsmittel und Wasser verstanden. Beispielhaft seien für organische Lösungsmittel gegebenenfalls halogenierte aliphatische oder aromatische Kohlenwasserstoffe, wie Pentan, Hexan, technisches Hexan, Heptan, Benzol, Toluol, Xylol, technisches Xylol, Methylenchlorid, Chloroform, Ether, wie Dimethylether, Diethylether, THF, Dioxan, Diglykol, Dimethyldiglykol(Diglyme), Alkohole, wie Methanol, Ethanol, Propanol Ketone, wie Aceton, Ester, wie Essigsäurethylester, Nitrile, wie Acetonitril, oder Carboxamide, wie Dimethylformamid (DMF), Dimethylacetamid (DMA), N-Methylpyrrolidon (NMP) verstanden.

Bevorzugt sind organische Lösungsmittel mit Metall-komplexierenden Eigenschaften, also organische Lösungsmittel, die Gruppen oder Atome mit Elektronen-Donor-Eigenschaften enthalten, wie NMP, DMF oder DMA.

Aufgrund der höheren Zündtemperatur ihrer Mischungen mit nachstehenden Basen, insbesondere Tripropylamin, sind DMF, DMA oder NMP als organische Lösungsmittel besonders bevorzugt.

Unter Basen werden Brönsted-Basen wie LiOH, NaOH, KOH, Li-, Na- oder K-Alkoholate oder quartäres Ammoniumhydroxid oder C₂-C₁₄-Dialkylamine, wie Dimethylamin, Diethylamin, Methylethylamin, Dipropylamin, Diisopropylamin, Ethylbutylamin, Dibutylamin oder C₃-C₂₀-Trialkylamine, wie Trimethylamin, Triethylamin, Tripropylamin oder Tributylamin verstanden.

Bevorzugte Basen sind C₃-C₂₀-Trialkylamine.

Aufgrund der höheren Zündtemperatur der Mischung mit DMF oder DMA ist die Verwendung von Tripropylamin als Base besonders bevorzugt.

Die Kombinationen Tripropylamin als Base und DMF oder DMA als Lösungsmittel stellen deshalb eine bevorzugte Ausführungsform dar.

Im erfindungsgemäßen Verfahren beträgt die Konzentration des Edukts β-IP typischerweise 0,5 bis 4,0 mol/l, insbesondere 2,5 bis 3,6 mol/l.

Die Temperatur der Reaktion wird typischerweise auf 20°C geregelt, um die stark exotherme Reaktion kontrolliert ablaufen zu lassen.

Die Konzentrationen der Reagenzien und Katalysatoren sind nicht kritisch und betragen typischerweise für den Katalysator 0,1 mol-% bis 5 mol-%, insbesondere 0,3 mol-% bis 0,7 mol-% und für die Base 0,1 mol/l bis 0,5 mol/l, insbesondere 0,15 mol/l bis 0,35 mol/l.

Die Dauer der Umsetzung beträgt typischerweise 0,1 bis 10 Stunden, insbesondere 4 bis 8 Stunden.

Um eine weitere Erhöhung der Konzentration an β-IP und damit eine weitere Erhöhung der Raum-Zeit-Ausbeute zu ermöglichen, wird in einer besonders bevorzugten Ausführungsform das Verfahren unter inverser Reaktionsführung, d. h. unter langsamer, kontinuierlicher Zuführung von β-IP in das Reaktionsgemisch durchgeführt. Typischerweise beträgt die Dauer der Zuführung zwischen einer und acht Stunden, insbesondere 4 Stunden.

Das Verfahren zeichnet sich gegenüber dem Stand der Technik durch folgende Vorteile aus:
- Das Verfahren ist auch im großtechnischen Maßstab wirtschaftlich, d.h. mit hohen Raum-Zeit-Ausbeuten durchführbar.
- Es werden auch bei hohen Eduktkonzentrationen an β-IP (3,5 mol/l) bei einem Umsatz von 100 % Selektivitäten und damit auch Ausbeuten von bis zu 90 % erreicht. Dadurch wird die Raumzeit-Ausbeute erhöht. Die Selektivitätserhöhung gelingt durch eine Reduzierung der normalerweise schwierig abzutrennenden Nebenprodukte IV-VII.
- Durch geeignete Wahl der Base und des Lösungsmittels kann die Zündtemperatur des Reaktionsgemisches erhöht werden, was einen weiteren sicherheitstechnischen Vorteil bietet.

Die nachstehenden Beispiele erläutern die Erfindung.

### Beispiele 1-3

### Herstellung der Katalysatoren

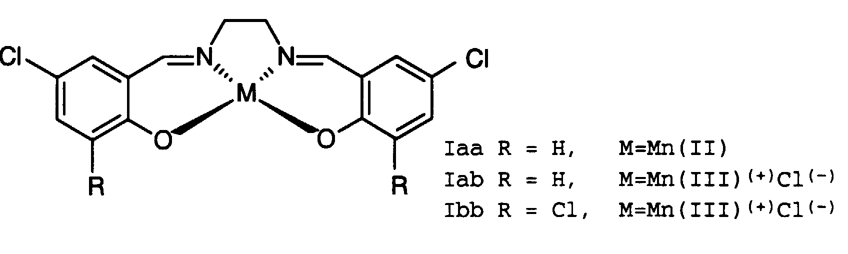

### Beispiel 1

### Herstellung des Katalysators der Formel Iaa

6,75 g (20 mmol) der Schiff'schen Base (Salenligand, hergestellt aus Ethylendiamin und 5-Chlor-2-Hydroxy-Benzaldehyd) wurden unter Stickstoff in Natronlauge (1,6 g NaOH auf 100 ml Wasser) bei 80 bis 85°C gelöst. Zu dieser Lösung tropfte man innerhalb von 30 Minuten eine Lösung aus 3,38 g (20 mmol) MnSO₄.H₂O in 15 ml Wasser und rührte bis zum vollständigen Umsatz der Schiff'schen Base (ca. 2 bis 3 h; DC-Kontrolle; Cyclohexan/Essigester 2:1) bei 85°C nach.

Die Reaktionsmischung wurde auf 10°C gekühlt, der orange-braune Niederschlag abfiltriert und mit Wasser neutralisiert. Das Produkt wurde bis zur Gewichtskonstanz bei 50°C im Trockenschrank getrocknet.
- Ausbeute:: 7,4 - 7,7 g (94 bis 99 % der Theorie)

### Beispiel 2

### Herstellung des Katalysators der Formel Iab

6,75 g (20 mmol) der entsprechenden Schiff'schen Base (Salenligand, hergestellt aus Ethylendiamin und 5-Chlor-2-Hydroxy-Benzaldehyd) wurden in 150 ml siedendem Ethanol gelöst, mit 5,36 g Mn(OAc)₃.2 H₂O (20 mmol) vesetzt und drei Stunden unter Rückfluß gehalten. Anschließend wurden drei Äquivalente Lithiumchlorid (2,54 g, 60 mmol) zugegeben und die Reaktionslösung weitere drei Stunden gekocht. Nach Abkühlen auf Raumtemperatur wurde der braunschwarze Feststoff abfiltriert und mit MTB-Ether (100 ml) gewaschen. Bei 50°C wird der Komplex über Nacht im Vakuum getrocknet.
- Ausbeute:: 8,40 g (99 % der Theorie)

### Beispiel 3

### Herstellung des Katalysators der Formel Ibb

Herstellung analog zu Beispiel 3, mit dem Unterschied, daß ein Salenligand, hergestellt aus Ethylendiamin und 3,5-Dichlor-2-Hydroxy-Benzaldehyd verwendet wurde.
- Ausbeute:: 95 bis 99 % der Theorie

### Beispiele 4 bis 8

### Herstellung von OIP durch Oxidation von β-IP in Gegenwart der Katalysatoren Iaa, Iab, und Ibb und verschiedener Lösungsmittel

520 ml DMF bzw. DMA (Dimethylacetamid), 28,6 g Tripropylamin (0,2 mol) und etwa ein Drittel der benötigten Menge an Katalysator (total: je 9 mmol, 0,45 mol-%) wurden in einen 1-L-HWS-Glasreaktor vorgelegt und auf 20°C temperiert. Unter Sauerstoffbegasung (mit Abgasführung) wurden innerhalb von 4 Stunden unter Temperaturkontrolle (20 +/- 1°C) 276 g β-Isophoron (2,0 mol) zugetropft. Die restliche Menge des Katalysators wurde in DMF bzw. DMA suspendiert und nach zwei, vier bzw. sechs Stunden portionsweise zudosiert. Die Gesamtbegasung betrug acht Stunden.

Die Ausbeuten an Oxoisophoron wurden gaschromatographisch unter Verwendung eines internen Standards (Methylbenzoat) bestimmt. Neben der Ausgangsverbindung β-Isophoron und dem Produkt Oxoisophoron wurde die Menge der entstandenen Nebenprodukte IV, V, VI und VII gaschromatographisch durch Flächenintegration bestimmt.

Die Reaktion wurde mit verschiedenen Katalysatoren und Lösungsmitteln durchgeführt. Die jeweiligen Reaktionsbedingungen und Ergebnisse sind in Tabelle 1 zusammengestellt.

### Vergleichsbeispiele 1 und 2

### Herstellung von OIP in Gegenwart der Katalysatoren IIa, IIb und III

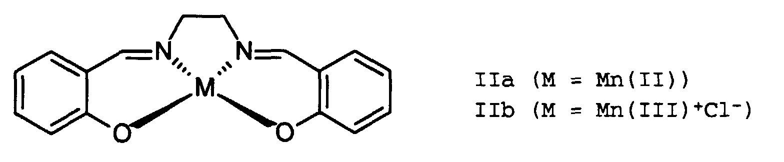

Die Herstellung von OIP erfolgte analog zu den Beispielen 5 bis 13 unter Verwendung der Katalysatoren IIa, IIb in DMF als Lösungsmittel. Die Reaktionsbedingungen und Ergebnisse sind in Tabelle 2 zusammengefaßt.

**Tabelle 1**

| Beispiel | Katalysator | Lösungsmittel | Ausbeute in [%] | | | | | |
|---|---|---|---|---|---|---|---|---|
| | | | OIP | β-IP | α-IP (IV) | V | VI | VII |
| 4 | Iaa | DMF | 86,2 | 0 | 1,6 | 1,9 | 0,3 | 1,7 |
| 5 | Iaa | DMA | 90,0 | 0 | 1,5 | 1,4 | 0,2 | 0,7 |
| 6 | Iab | DMF | 85,5 | 0 | 1,8 | 1,6 | 0 | 0,9 |
| 7 | Iab | DMA | 88,6 | 0 | 1,7 | 1,5 | 0 | 1,9 |
| 8 | Ibb | DMF | 89,3 | 0 | 1,8 | 1,9 | 0 | 1,2 |

**Tabelle 2**

| Beispiel | Katalysator | Lösungsmittel | Ausbeute in [%] | | | | | |
|---|---|---|---|---|---|---|---|---|
| | | | OIP | β-IP | α-IP (IV) | V | VI | VII |
| 1 | IIa | DMF | 82,4 | 0,3 | 1,9 | 4,4 | 0,5 | 1,2 |
| 2 | IIb | DMF | 81,2 | 0 | 3,2 | 3,8 | 2,9 | 2,8 |

## Patentansprüche

1. Verfahren zur Herstellung von 3,5,5-Trimethylcyclohex-2-en-1,4-dion durch Oxidation von 3,5,5-Trimethylcyclohex-3-en-1-on mit molekularem Sauerstoff in Gegenwart eines Lösungsmittels, einer Base und einem Katalysator, dadurch gekennzeichnet, daß man als Katalysator einen Mangansalenkomplex der allgemeinen Formel I 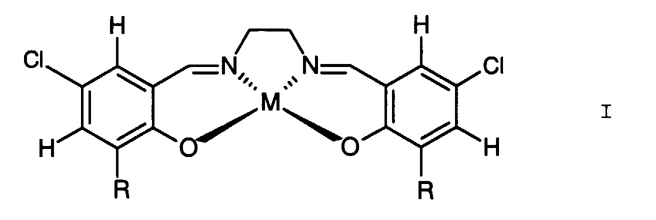 verwendet, wobei
R Wasserstoff oder Cl und
M Mn(II) oder Mn(III) ⁽⁺⁾Cl⁽⁻⁾ bedeuten.

2. Verfahren nach Anspruch 1, dadurch gekennzeichnet, daß man als Lösungsmittel ein Lösungsmittel mit komplexierenden Eigenschaften verwendet.

3. Verfahren nach Anspruch 1, dadurch gekennzeichnet, daß man als Lösungsmittel Dimethylformamid (DMF), N-Methylpyrrolidon (NMP) oder Dimethylacetamid (DMA) verwendet.

4. Verfahren nach einem der Ansprüche 1 bis 3, dadurch gekennzeichnet, daß man als Base ein Trialkylamin mit 3 bis 20 C-Atomen verwendet.

5. Verfahren nach einem der Ansprüche 1 bis 3, dadurch gekennzeichnet, daß man als Base Tripropylamin verwendet.

6. Verfahren nach Anspruch 1, dadurch gekennzeichnet, daß man als Lösungsmittel Dimethylformamid (DMF) oder Dimethylacetamid (DMA) in Kombination mit Tripropylamin als Base verwendet.

7. Verfahren nach Anspruch 1, dadurch gekennzeichnet, daß man das Verfahren unter inverser Reaktionsführung durch Zuleiten des Edukts zur Reaktionsmischung, enthaltend das Lösungsmittel, die Base und den Katalysator, durchführt.
